# EUROPÄISCHE PATENTSCHRIFT

(11) **EP 2 504 335 B1**
(45) Veröffentlichungstag und Bekanntmachung des Hinweises auf die Patenterteilung: **04.09.2013**
(21) Anmeldenummer: 10781894.0
(22) Anmeldetag: 22.11.2010
(51) Int. Cl.: C07D 471/04

(54) **Verfahren zur Reinigung von Methyl-{4,6-diamino-2-[1-(2-fluorobenzyl)-1H-pyrazolo[3,4-b]pyridin-3-yl]pyrimidin-5-yl}-carbamat**
Process for the purification of methyl-{4,6-diamino-2-[1-(2-fluorobenzyl)-1H-pyrazolo[3,4-b]pyridin-3-yl]pyrimidin-5-yl}-carbamate
Procédé de purification du {4,6-diamino-2-[1-(2-fluorobenzyl)-1H-pyrazolo[3,4-b]pyridin-3-yl]pyrimidin-5-yl}-carbamate de méthyle

(30) Priorität: 27.11.2009 EP 09177369
(43) Veröffentlichungstag der Anmeldung: 03.10.2012
(73) Patentinhaber: Bayer Intellectual Property GmbH, 40789 Monheim (DE)
(72) Erfinder: MAIS, Franz-Josef, 40591 Düsseldorf (DE); REHSE, Joachim, 42799 Leichlingen (DE); JOENTGEN, Winfried, 50735 Köln (DE); SIEGEL, Konrad, 40597 Düsseldorf (DE)
(74) Vertreter: BIP Patents
(86) Internationale Anmeldenummer: PCT/EP2010/067884
(87) Internationale Veröffentlichungsnummer: WO 2011/064156

(56) Entgegenhaltungen:
- WO-A1-03/095451
- WO-A1-2005/046725
- WO-A1-2008/031513
- WO-A1-2010/079120
- CHEMMEDCHEM, Bd. 4, Nr. 5, Mai 2009 (2009-05), Seiten 853-865, XP002622814, ISSN: 1860-7179, DOI: DOI:10.1002/CMDC.200900014 in der Anmeldung erwähnt
- EVANS R ET AL: "The Preparation of 4-Amino- and Other Pteridines", JOURNAL OF THE CHEMICAL SOCIETY, CHEMICAL SOCIETY, LETCHWORTH; GB, 1. Januar 1956 (1956-01-01), Seiten 4106-4113, XP009144631, ISSN: 0368-1769
- SCHWOCH S ET AL: "2,3-DIHYDROSPIRO[1H-4- AND 5-AZABENZIMIDAZOLE-2.1'-CYCLOHEXANE] (= SPIRO[CYCLOHEXANE-1,2'(3'H)-1'H-IMIDAZO[4, 5-B]PYRIDINE] AND SPIRO[CYCLOHEXANE-1,2'(3'H)-1'H-IMIDAZO[4, 5-C] PYRIDINE] ): REACTIONS WITH NUCLEOPHILES", HELVETICA CHIMICA ACTA, VERLAG HELVETICA CHIMICA ACTA, BASEL, CH, Bd. 77, Nr. 8, 1. Januar 1994 (1994-01-01) , Seiten 2175-2190, XP002073789, ISSN: 0018-019X, DOI: DOI:10.1002/HLCA.19940770811
- BARRACLOUGH P ET AL: "Mono-arylation of 2,3- and 3,4-diaminopyridine and 4,5-diaminopyrimidine, and syntheses of putative inotrope/beta-adrenoceptor antagonists", JOURNAL OF CHEMICAL RESEARCH, SCIENCE REVIEWS LTD, GB, Bd. 9, Nr. 9, 1. Januar 1996 (1996-01-01), Seiten 2316-2335, XP002103810, ISSN: 0308-2342

## Beschreibung

Die vorliegende Erfindung betrifft Verfahren zur Reinigung von Methyl-{4,6-diamino-2-[1-(2-fluorobenzyl)-1H-pyrazolo[3,4-b]pyridin-3-yl]pyrimidin-5-yl}carbamat, d.h. der Verbindung der Formel (I)

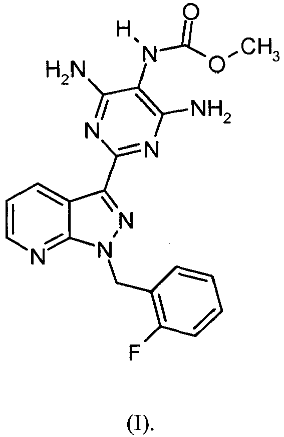

*Bei dem* Verfahren zur Reinigung des Rohproduktes der Formel (I) für die Verwendung als pharmazeutisch wirksamer Stoff wird. Methyl-{4,6-diamino-2-[1-(2-fluorobenzyl)-1H-pyrazolo[3,4-b]pyridin-3-yl]pyrimidin-5-yl}carbamat-sulfinyldimethan (1:2), d.h. eine Verbindung der Formel (II) als Zwischenstufe isoliert oder als Zwischenstufe in diesem Reinigungsverfahren, gegebenenfalls in einem Gemisch vorliegend, erzeugt wird

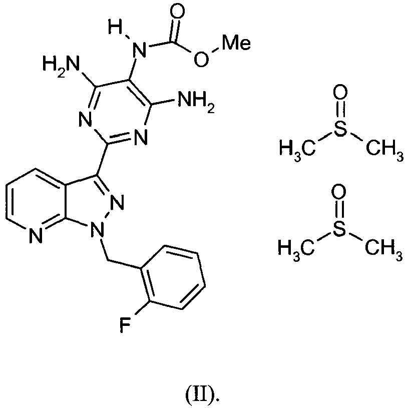

Die Verbindung der Formel (I) wirkt als Stimulator der löslichen Guanylatcyclase und kann als Mittel zur Prophylaxe und/oder Behandlung von kardiovaskulären Erkrankungen wie beispielsweise zur Behandlung des Bluthochdrucks und der Herzinsuffizienz, stabiler und instabiler Angina pectoris, peripheren und kardialen Gefäßerkrankungen, von Arrhythmien, zur Behandlung von thromboembolischen Erkrankungen und Ischämien wie Myokardinfarkt, Hirnschlag, transistorischen und ischämischen Attacken, peripheren Durchblutungsstörungen, Verhinderung von Restenosen wie nach Thrombolysetherapien, percutan transluminalen Angioplastien (PTA), percutan transluminalen Koronarangioplastien (PTCA), Bypass sowie zur Behandlung von Arteriosklerose, asthmatischen Erkrankungen und Krankheiten des Urogenitalsystems wie beispielsweise Prostatahypertrophie, erektiler Dysfunktion, weiblicher sexueller Dysfunktion, Osteoporose, Glaukom, pulmonaler Hypertonie, Gastroparese und Inkontinenz eingesetzt werden.

Die Herstellung der Verbindung der Formel (I) und deren Reinigung sind grundsätzlich bekannt. In WO 03/095451 wird die Herstellung der Verbindung der Formel (I) auf folgendem Weg beschrieben.

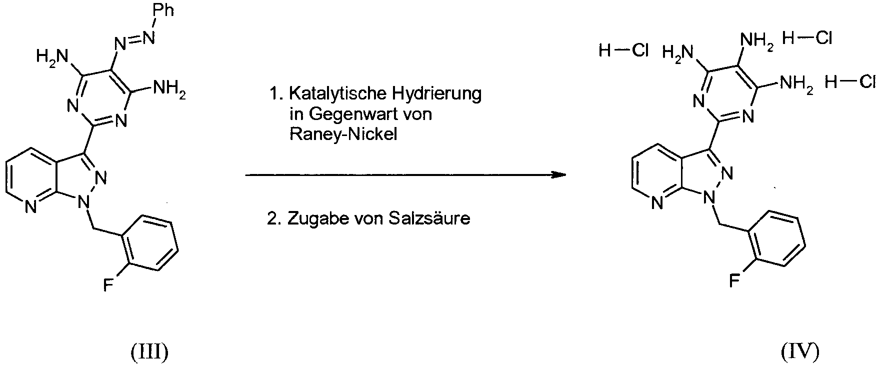

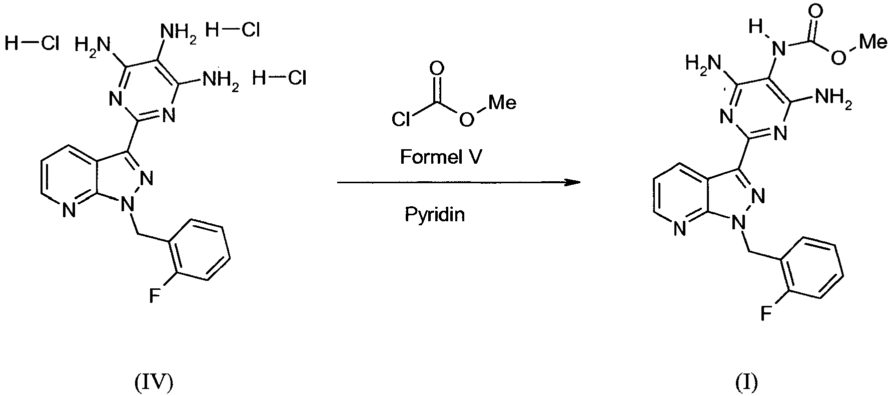

Dabei wird zunächst t 2-[1-(2-Fluorbenzyl)-1H-pyrazolo[3,4-b]pyridin-3-yl]-5-[(E)-phenyldiazenyl]pyrimidin-4,6-diamin der Formel (III) durch katalytische Hydrierung gespalten und die resultierende Trisaminoverbindung wird als 2-[1-(2-Fluorbenzyl)-1H-pyrazolo[3,4-b]pyridin-3-yl]-4,5,6-pyrimidintriamin Trihydrochlorid der Formel (IV) isoliert. Dieses Trihydrochlorid wird dann mit Chlorameisensäuremethylester der Formel (V) in Pyridin als Lösungsmittel zu Methyl-4,6-diamino-2-[1-(2-fluorbenzyl)-1H-pyrazolo[3,4-b]pyridin-3-yl]-5-pyrimidinylcarbamat der Formel (I) umgesetzt. Alternativ wird in ChemMedChem 2009, 4, 853-865 beschrieben, daß die Trisaminoverbindung als Trihydrochlorid isoliert wird und anschließend die HCl freie Base durch Ausschütteln mit wäßriger NaHCO₃-Lösung erzeugt und die freie Base mit dem Chlorameisensäuremethylester der Formel (V) zur Verbindung der Formel (I) in Pyridin als Lösungsmittel umgesetzt wird.

Diese Synthese besitzt eine Reihe von Nachteilen, die für eine technische Durchführung in großem Maßstab sehr ungünstig sind. Dies gilt vor allem für die Isolierung der Trisaminoverbindung als Trihydrochlorid. Die Zugabe der Salzsäure verlangt eine säurefeste technische Anlage und die Ausbeute des Schrittes ist nur unbefriedigende 59,3% d.Th. (siehe Beispiel 8A von WO 03/095451 Auch die Durchführung der Umsetzung der Trisaminoverbindung der Formel (IV) oder der entsprechenden HCl-freien Base in Pyridin als Lösungsmittel ist nachteilig. Die Verbindung der Formel (I) kann nur durch technisch unvorteilhaftes vollständiges Eindampfen der Reaktionsmischung isoliert werden (siehe z.B. Beispiel 5 von WO 03/095451). Solche Schritte führen in größerem Maßstab in der Regel zu erheblichen Problemen wie Anbackungen oder thermische Zersetzung aufgrund der im größeren Maßstab wesentlich längeren thermischen Belastung. Erheblich nachteilig ist auch, daß nach z.B. Beispiel 5 vom WO 03/095451 das Produkt durch Auskochen in Diethylether gereinigt wird. Dieser Schritt kann aufgrund der leichten Entzündlichkeit von Diethylether nur unter erhöhtem technischem Aufwand durchgeführt werden. Besonders nachteilig ist jedoch, daß die Substanz der Formel (I) die nach WO 03/095451 hergestellt wurde noch eine Reihe von Verunreinigungen in Mengen enthält, die einer Verwendung als pharmazeutischem Wirkstoff entgegen stehen.

Es bestand daher Aufgabe ein vereinfachtes Verfahren zu finden, das sicher und auch im großtechnischen Maßstab durchgeführt werden kann und gleichzeitig einen Wirkstoff höchster Reinheit im pharmazeutisch akzeptabler Qualität liefert.

Es wurde nun ein Verfahren zur Reinigung von Methyl-{4,6-diamino-2-[1-(2-fluorobenzyl)-1H-pyrazolo[3,4-b]pyridin-3-yl]pyrimidin-5-yl}carbamat Formel (I) 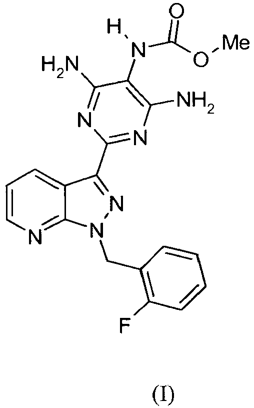 für seine Verwendung als pharmazeutischer Wirkstoff gefunden.

Dieses neue Verfahren unterscheidet sich von den bislang bekannten Verfahren im folgenden Punkten:
- Die Reinigung des Rohproduktes der Formel (I) für die Verwendung als pharmazeutischer Wirkstoff erfolgt über die Verbindung Methyl-{4,6-diamino-2-[1-(2-fluorobenzyl)-1H-pyrazolo[3,4-b]pyridin-3-yl]pyrimidin-5-yl}carbamat-sulfinyldimethan (1:2), d.h. eine Verbindung der Formel (II) als isolierte Zwischenstufe oder welche in einem Gemisch erzeugt wird

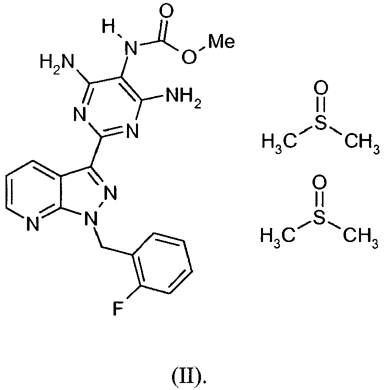

DieserUntersched ermöglicht die Überwindung der Nachteile der bislang bekannten Verfahren und einen Wirkstoff in hoher Ausbeute und hoher Reinheit in pharmazeutisch akzeptabler Qualität.

Im folgenden wird das erfindungsgemäße Verfahren zur Reinigung der Verbindung der Formel (I) über das Zwischenprodukt der Formel (II) genau beschrieben.

### Reinigung des Rohproduktes der Verbindung der Formel (I)

Das Rohprodukt der Formel (I) wird für die Verwendung als pharmazeutischer Wirkstoff gereinigt. Bei dieser Reinigung wird die Verbindung der Formel (II) als Zwischenprodukt erzeugt.

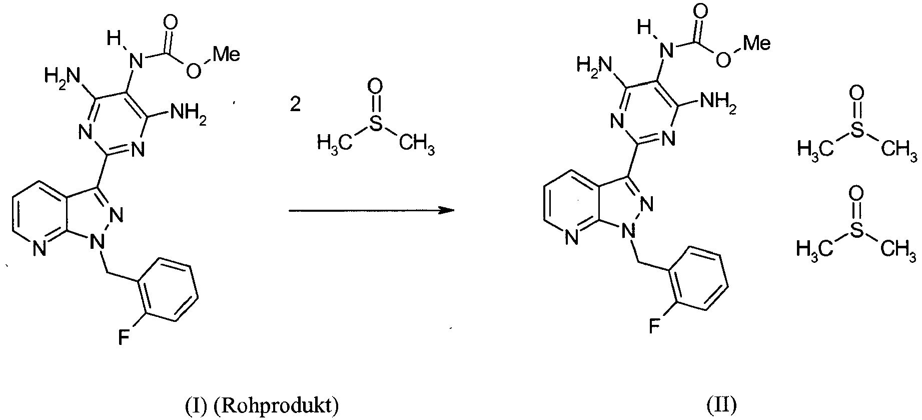

Dazu wird das Rohprodukt der Formel (I) in DMSO gegebenenfalls in Gegenwart eines pharmazeutisch akzeptablen einfachen Lösungsmittels aus der Klasse der Ketone, Ether, Ester oder Alkohole gelöst. Als Beispiele für solche Lösungsmittel seien genannt: Methanol, Ethanol, Isopropanol, 1-Butanol, 2-Butanol, Ethylacetat, Isopropyl- oder Propylacetat, Butylacetat, tert.-Butylmethylether, Diisopropylether, Aceton, Methylethylketone, Methylisobutylketon usw. Bevorzugt sind Ethanol, Isopropanol, Ethylacetat, Isopropylacetat, Butylacetat, Methylethylketon, Methylisobutylketon, besonders bevorzugt ist Ethylacetat.

Es können auch Gemische dieser Lösungsmittel eingesetzt werden

DMSO wird zugegeben in einer Menge von 250 bis 750 Gew.-% auf die Menge des Rohproduktes der Formel (I), bevorzugt 350 bis 600 Gew.-%.

Gegebenenfalls kann zu dieser Mischung Aktivkohle zugesetzt werden in einer Menge von 0,25 bis 25 Gew.-% auf die die Menge des Rohproduktes der Formel (I), bevorzugt 0,5 bis 10% Gew.-%.

Zur Erzeugung einer Lösung wird das Gemisch erhitzt auf 40-120°C, bevorzugt auf 50-100°C.

Zur Erzeugung eines pharmazeutisch akzeptablen Produktes muß die Lösung filtriert werden. Die Filtration muß unabhängig davon durchgeführt werden ob Aktivkohle zugesetzt wurde oder nicht.

Die Menge des vor der Filtration zugesetzten pharmazeutisch akzeptablen Lösungsmittels beträgt 50 bis 200 Gew. % bezogen auf DMSO, bevorzugt 75 bis 150 Gew.-%.

Die Filtration wird in der Hitze durchgeführt, die Temperaturen sind 40-120°C, bevorzugt 50-100°C.

Nach der Filtration wird in der Hitze ein pharmazeutisch akzeptables Lösungsmittel zugesetzt, bevorzugt der gleiche Lösungsmittel wie zuvor.

Die Gesamtmenge Menge des vor und nach der Filtration zugesetzten Lösungsmittels beträgt 125 bis 300 Gew.-% bezogen auf DMSO, bevorzugt 150-250 Gew.-%.

Die Temperatur der Zugabe liegt bei 30-100°C, bevorzugt 35-75°C.

Vor der Isolierung des gereinigten Feststoffes der Formel (II) wird zur Vervollständigung der Fällung abgekühlt in einen Temperaturbereich von 0-30°C, bevorzugt auf Normaltemperatur von z.B. 20-25°C.

Die Isolierung wird über übliche Isolieraggregate wie Nutsche oder Zentrifuge durchgeführt. Zur Entfernung der Mutterlauge wird das Produkt bei der Isolierung mit einem pharmazeutisch akzeptablen Lösungsmittelgewaschen, bevorzugt ist das gleiche Lösungsmittel wie zuvor. Das so erhaltene Produkt der Formel (II) kann nun getrocknet werden oder auch in feuchter Form mit Gehalt an Restlösungsmittel in eine Auskochung eingesetzt werden.

Das Produkt nach der DMSO-Umlösung enthält auch nach der Wäsche noch erhebliche Mengen an DMSO. Der Gehalt an DMSO liegt in Abhängigkeit von der Güte der Wäsche üblicherweise bei 26 bis 35 Gew.-%. Bei sehr gut gewaschenen Produkten liegt der Gehalt an DMSO bei 27-31 Gew.-%. Der Rest zu 100% ist nahezu ausschließlich das Produkt der Formel (I). Damit entspricht die Zusammensetzung des aus der DMSO-Umlösung erhaltenen Feststoffs einem DMSO-Disolvat der Verbindung der Formel (I), d.h. einer der Struktur der Formel (II).

Es ist erfindungsgemäß besonders bevorzugt bei der Reinigung der Verbindung der Formel (I) das DMSO-haltige Produkt, das die Zusammensetzung der Verbindung der Formel (II) hat als Feuchtprodukt oder in im Vakuum getrockneter Form zu isolieren.

Die Verbindung der Formel (II) ist neu. Sie kann wie in bei den folgenden Ausführungsbeispielen beschrieben ist, in reiner Form hergestellt werden und analytisch charakterisiert werden.

Für eine pharmazeutische Verwendung muß aus dem DMSO-haltigen Produkt der Formel (II) das DMSO entfernt werden.

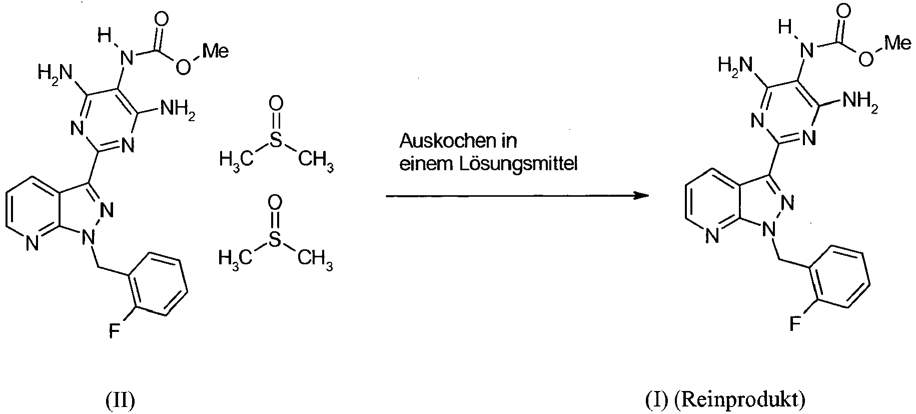

Dazu wird das Produkt der Formel (II) in einem pharmazeutisch akzeptablen Lösungsmittel aus der Klasse der Ketone, Ether, Ester oder Alkohole gelöst. Als Beispiele für solche Lösungsmittel seien genannt: Methanol, Ethanol, Isopropanol, 1-Butanol, 2-Butanol, Ethylacetat, Isopropyl- oder Propylacetat, Butylacetat, tert.-Butylmethylether, Diisopropylether, Aceton, Methylethylketone, Methylisobutylketon usw. Bevorzugt sind Ethanol, Isopropanol, Ethylacetat, Isopropylacetat, Butylacetat, Methylethylketon, Methylisobutylketon. Es können auch Gemische dieser Lösungsmittel eingesetzt werden. Besonders bevorzugt ist Ethylacetat oder ein Gemisch aus Ethylacetat mit Ethanol.

Die Auskochung wird am Rückfluß des jeweiligen Lösungsmittels oder gegebenenfalls bei leicht erhöhtem Drück durchgeführt. Die Temperatur ist 50-150°C, bevorzugt 70-120°C.

Das erfmdungsgemäße Verfahren bietet deutliche Vorteile gegenüber dem Stand der Technik. Vor allem überraschend ist, daß die Reinigung für eine pharmazeutische Verwendung besonders aus einer Umlösung mit einem DMSO-haltigen Lösungsmittel erfolgt und daß die neue Verbindung der Formel (II) als gereinigtes Produkt erhalten wird. Durch diesen Schritt werden sämtliche Verunreinigungen bis auf niedrige Restmengen abgetrennt, so daß man nach der Entfernung des DMSO durch einfaches Auskochen einen hochreinen Feststoff zurück behält. Dieser Feststoff ist der Regel farblos bis ganz leicht gelb und die analytische Reinheit (HPLC) liegt deutlich über 99 Gew.-% was für eine pharmazeutische Verwendung sehr vorteilhaft ist.

Das Verfahren ist technisch sicher durchführbar und es erlaubt eine Produktion im großtechnischen Maßstab. Es läßt sich flexibel an betriebliche apparative Voraussetzungen anpassen.

Eine weitere besonders bevorzugte Ausführungsform ist, daß bei der Reinigung des Rohproduktes der Formel (I) die Zwischenisolierung des Produktes der Formel (II) in einem Nutschtrockner durchgeführt wird. Die folgende Entfernung des DMSO aus dem im Nutschtrockner zwischenisolierten Produkt der Formel (II) erfolgt durch direkte Zugabe von Lösungsmittel in den Nutschtrockner mit oder ohne Zwischentrocknung des Produktes der Formel (II). Dadurch wird ein offenes Handling des Feststoffs des Produktes (II) mit der Gefahr von Verunreinigung vermieden.

### Experimenteller Teil

### Abkürzunzen und Akronyme:

| | |
|---|---|
| abs. | absolut |
| cat. | katalytisch |
| CI | chemische Ionisation (bei MS) |
| d | Tag(e) |
| DC | Dünnschichtchromatographie |
| DMF | Dimethylformamid |
| DMSO | Dimethylsulfoxid |
| d. Th. | der Theorie (bei Ausbeute) |
| ee | Enantiomerenüberschuss |
| EI | Elektronenstoß-Ionisation (bei MS) |
| ent | Enantiomer / enantiomerenrein |
| eq | Äquivalent(e) |
| ESI | Elektrospray-Ionisation (bei MS) |
| GC-MS | Gaschromatographie-gekoppelte Massenspektrometrie |
| Gew.-% | Gewichtsprozent |
| h | Stunde(n) |
| HPLC | Hochdruck-, Hochleistungsflüssigchromatographie |
| konz. | konzentriert |
| LC-MS | Flüssigchromatographie-gekoppelte Massenspektrometrie |
| min | Minute(n) |
| MS | Massenspektrometrie |
| NMR | Kernresonanzspektrometrie |
| Ph | Phenyl |
| R_{f} | Retentionsindex (bei DC) |
| Rₜ | Retentionszeit (bei HPLC) |
| RT | Raumtemperatur |
| v/v | Volumen-zu-Volumen-Verhältnis (einer Lösung) |
| wässr. | wässrig, wässrige Lösung |

### Die folgenden Beispiele erläutern die Erfindung ohne Sie jedoch darauf einzuschränken. Referenz-

### Beispiel 1

### Herstellung von 2-[1-(2-Fluorbenzyl)-1H-pyrazolo[3,4-b]pyridin-3-yl]-4,5,6-pyrimidintriamin (V1)

In einem Druckautoklaven wurden 1100g der Verbindung der Formel (III) in 5,41 DMF suspendiert. Man gab 44g eines handelsüblichen wasserfeuchten (ca. 50%ig) 5%Pd-Kohle Katalysators zu und der verschlossene Autoklav wurde nach Inertisierung mit Stickstoff und Aufdrücken von Wasserstoff ca. 18h bei 65bar Wasserstoff und ca. 60°C Innentemperatur hydriert. Nach dem Abkühlen auf ca. 25°C, Entspannen und Inertisieren wurde der Autoklaveninhalt ausgenommen, wobei mit 650ml DMF nachgespült wurde.

Drei solcher gleichartig durchgeführter Ansätze wurden vereinigt, der Altkatalysator wurde abfiltriert, man spülte mit 1,11 DMF und das Filtrat wurde im Vakuum auf ca. ein Drittel seiner Masse eingeengt. In den Rückstand von ca. 6,5kg wurden nacheinander 8,251 Methanol und 8,251 Wasser eindosiert, die Suspension wurde zur Vervollständigung der Kristallisation auf ca. 5°C abgekühlt, der Feststoff wurde abfiltriert und mit Methanol/Wasser (1:1 Vol) gewaschen. Das Produkt wurde bei 50°C im Vakuum getrocknet. Die Auswaage betrug 2415g entsprechend 91,8% d.Th. Der Gehalt des Zielproduktes der Formel (VI) (freie Base) betrug >98 Flächen-% bzw. >97 Gew.-%. Die größten Verunreinigungen waren DMF (ca. 0,8 Gew. %) und Wasser (ca. 0,5 Gew.-%).

### Referenz- Beispiel 2

### Herstellung des Rohproduktes von Methyl-4,6-diamino-2-[1-(2-fluorbenzyl)-1H-pyrazolo[3,4-b]pyridin-3-yl]-5-pyrimidinylcarbamat (I)

In einem Reaktionsgefäß wurden 3063g der Verbindung der Formel (VI) und 30,71 techn. Isopropanol vorgelegt. Dazu dosierte man unter Rühren 1641g Dimethyldicarbonat bei 20-25°C ein und rührte 22h bei dieser Temperatur nach. Das ausgefallene Produkt wurde abgesaugt, mit technischem Isopropanol gewaschen und im Vakuum bei 95°C getrocknet. Man erhielt eine Auswaage von 3748g bzw. 105,9% d.Th. Das Rohprodukt der Formel (I) enthielt unter anderem ca. 4,7% praktisch nicht durch Trocknung entfernbares Isopropanol (es lag teilweise ein Isopropanol-Solvat vor) und der analytische Gehalt lag bei 89,5 Gew.-% (HPLC). Bezogen auf diesen Gehalt lag die Ausbeute bei 94,8% d.Th.

### Referenz-Beispiel 3

### Herstellung von 2-[1-(2-Fluorbenzyl)-1H-pyrazolo[3,4-b]pyridin-3-yl]-4,5,6-pyrimidintriamin (V1)

In einem Druckautoklaven wurden 300g der Verbindung der Formel (III), 1600ml DMF und 60g wasserfeuchtes Raney-Nickel vorgelegt und nach Inertisierung bei 60°C Innentemperatur, 65bar Wasserstoff für 18h hydriert. Nach Abkühlen und Entspannen wurde der Altkatalysator abfiltriert und mit 100ml DMF gespült. Das Filtrat wurde auf 530g im Vakuum eingeengt und zu dem Rückstand dosierte man bei 35-40°C 750ml Methanol und dann nach Abkühlen bei 0-5°C 750ml Wasser ein. Der Feststoff wurde filtriert und bei 50°C im Vakuum getrocknet. Die Auswaage an Feststoff der Formel (VI) (freie Base) betrug 219,7g bzw. 91,8% d.Th.

### Referenz- Beispiel 4

### Herstellung des Rohproduktes von Methyl-4,6-diamino-2-[1-(2-fluorbenzyl)-1H-pyrazolo[3,4-b]pyridin-3-yl]-5-pyrimidinylcarbamat (I)

In einem Reaktionsgefäß wurden 1,50kg der Verbindung der Formel (VI) in 14,251 Isopropanol vorgelegt und unter Rühren auf 35°C erhitzt. Dazu dosierte man 531g Chlorameisensäuremethylester in 30min gleichmäßig schnell ein, spülte mit 750ml Isopropanol nach und rührte 16h bei 35°C nach. Dann erhitzte man auf 50°C, dosierte 3,851 Methanol und 606g Triethylamin bei 50°C unter Rühren ein und spülte mit 450ml Methanol nach. Dann rührte man 1h bei 50°C nach, kühlte auf RT ab und rührte bei RT 1h nach. Der suspendierte Feststoff wurde abgesaugt, mit je 3,01 Isopropanol/Methanol (4:1) zweimal und einmal mit 3,01 Isopropanoll gewaschen und trocken gesaugt. Das Feuchtprodukt wurde bei 50°C für 1h und anschließend bei 100°C für 22h im Vakuumtrockenschrank getrocknet. Man erhielt eine Auswaage von 1,793kg bzw. 103,3% d.Th. Das Produkt der Formel (VI) enthielt 6,45% praktisch nicht durch Trocknung entfernbares Isopropanol (es lag teilweise ein Isopropanol-Solvat vor) und der analytische Gehalt lag bei 87,9 Gew.-% (HPLC). Bezogen auf diesen Gehalt lag die Ausbeute bei 90,8% d.Th.

### Beispiel 5

### Herstellung von Methyl-{4,6-diamino-2-[1-(2-fluorobenzyl)-1H-pyrazolo[3,4-b]pyridin-3-yl]pyrimidin-5-yl}carbamat-sulfinyldimethan (1:2) der Formel (II)

1230g eines analog Beispiel 2 hergestellten des Rohproduktes der Formel (I) (Gehalt 89,1%) wurden in 15,01 Ethylacetat und 6,61 DMSO am Rückfluß (ca. 85-87°C) gelöst, in der Hitze über einen feinporigen Filter filtriert und langsam unter Rühren auf RT und dann auf 10°C abkühlen lassen. Der ausgefallene Feststoff wurde abfiltriert, dreimal mit insgesamt 1,21 Ethylacetat gewaschen und im Vakuum bei 50°C für 20h getrocknet. Man erhielt 1382g Auswaage. Das sind unter Berücksichtigung des Gehaltes des Einsatzstoffes der Formel (I) 91,2% d.Th. Der Feststoff enthielt 27,4 Gew.-% (GC) DMSO und 72,6 Gew.-% (HPLC) an Verbindung der Formel (I). Er entsprach demnach analytisch einem DMSO-Bissolvat der Formel (II).
¹H-NMR (500MHz in DMF-d₇):
d = 2,58 (s, 12H, 4 CH₃ an DMSO), 3,65 (s, 3H, O-CH₃), 5,89 (s ,2H, -CH₂-), 6,33 (s, 4H, 2 -NH₂), 7,05-7,39 (m, 5H, 4 aromatische H am o-Fluorbenzyl Substituenten und 1H am Pyridoring meta zum Pyrido-Stickstoff), 8,0 (s, 1H, -NH-) 8,60 (dd, 1H, am Pyridoring ortho zum Pyrido-Stickstoff), 9,13 (dd, 1H, am Pyridoring para zum Pyrido-Stickstoff).

### Elementaranalyse:

| | | | |
|---|---|---|---|
| gefunden | C: 49,4% | berechnet | C: 48,92% |
| | H: 5,2% | | H: 5,18% |
| | N: 20,0% | | N: 19,84% |

### Beispiel 6

### Herstellung von reinen pharmazeutisch verwendbaren Methyl-4,6-diamino-2-[1-(2-fluorbenzyl)-1H-pyrazolo[3,4-b]pyridin-3-yl]-5-pyrimidinylcarbamat (I)

7,1kg des Produktes der Formel (II) wurden in 171,6kg Ethylacetat und 42kg Ethanol suspendiert und 20h bei Rückfluß (ca. 73-74°C Innentemperatur) gerührt. Die Suspension wurde auf RT abgekühlt, absaugt und vier Mal mit je 12,2kg Ethylacetat gewaschen. Danach wurde zwei Mal mit je 12,2kg Wasser zur Verdrängung des Ethylacetats gewaschen und das feuchte Produkt wurde bei 50°C im Vakuum bis zur Massekonstanz getrocknet. Die Ausbeute an reinem Produkt der Formel (I) betrug 4,3kg bzw. 84% d.Th. Der Gehalt des Produktes lag über 99°% (HPLC).

## Patentansprüche

1. Verfahren zur Reinigung von Methyl-{4,6-diamino-2-[1-(2-fluorobenzyl)-1H-pyrazolo[3,4-b]pyridin-3-yl]pyrimidin-5-yl}carbamat der Formel (I) **dadurch gekennzeichnet, dass** das Rohprodukt der Verbindung der Formel (I) in Dimethylsulfoxid gelöst und das dabei anfallende Methyl-{4,6-diamino-2-[1-(2-fluorobenzyl)-1H-pyrazolo[3,4-b]pyridin-3-yl]pyrimidin-5-yl}carbamat-sulfinyldimethan (1:2) der Formel 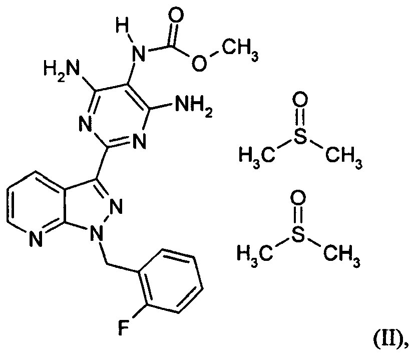 isoliert und das Dimethylsulfoxid anschliessend durch Auskochen in einem pharmazeutisch akzeptablen Lösungsmittel entfernt wird.

2. Methyl-{4,6-diamino-2-[1-(2-fluorobenzyl)-1H-pyrazolo[3,4-b]pyridin-3-yl]pyrimidin-5-yl}carbamat-sulfinyldimethan (1:2) der Formel

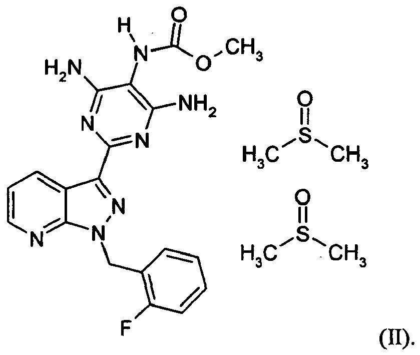

## Claims

1. Process for purifying methyl {4,6-diamino-2-[1-(2-fluorobenzyl)-1H-pyrazolo[3,4-b]pyridin-3-yl]pyrimidin-5-yl}carbamate of the formula (I), **characterized in that** the crude product of the compound of the formula (I) is dissolved in dimethyl sulphoxide and the resulting methyl {4,6-diamino-2-[1-(2-fluorobenzyl)-1H-pyrazolo[3,4-b]pyridin-3-yl]pyrimidin-5-yl}carbamate sulphinyldimethane (1:2) of the formula 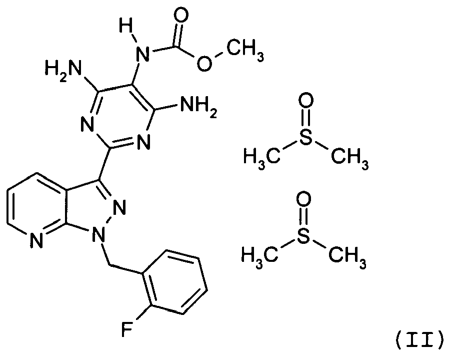 is isolated and the dimethyl sulphoxide is subsequently removed by boiling in a pharmaceutically acceptable solvent.

2. Methyl {4,6-diamino-2-[1-(2-fluorobenzyl)-1H-pyrazolo[3,4-b]pyridin-3-yl]pyrimidin-5-yl}carbamate sulphinyldimethane (1:2) of the formula

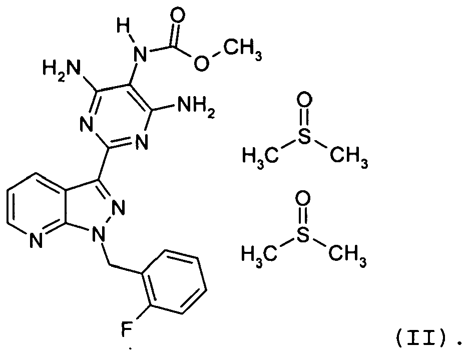

## Revendications

1. Procédé pour la purification de méthyl-{4,6-diamino-2-[1-(2-fluorobenzyl)-1H-pyrazolo[3,4-b]pyridin-3-yl]pyrimidin-5-yl)carbamate de formule (I), **caractérisé en ce qu'**on dissout le produit brut du composé de formule (I) dans du diméthylsulfoxyde et on isole le méthyl-{4,6-diamino-2-[1-(2-fluorobenzyl)-1H-pyrazolo[3,4-b]pyridin-3-yl]pyrimidin-5-yl}carbamate-sulfinyldiméthane (1:2) qui se forme de formule 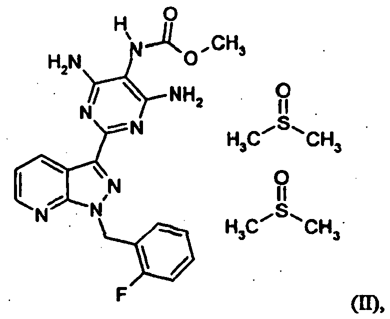 et on élimine ensuite le diméthylsulfoxyde par ébullition dans un solvant pharmaceutiquement acceptable.

2. Méthyl-{4,6-diamino-2-[1-(2-fluorobenzyl)-1H-pyrazolo[3,4-b]pyridin-3-yl]pyrimidin-5-yl}carbamate-sulfinyldiméthane (1:2) de formule
